# EUROPÄISCHE PATENTANMELDUNG

(11) **EP 3 028 738 A1**
(43) Veröffentlichungstag der Anmeldung: **08.06.2016**
(21) Anmeldenummer: 15003336.3
(22) Anmeldetag: 21.11.2015
(51) Int. Cl.: A61M 39/28, F16K 7/06

(54) **SCHLAUCHKLEMME**

(30) Priorität: 06.12.2014 DE 102014018164
(71) Anmelder: THOMAS MAGNETE GmbH, 57562 Herdorf (DE)
(72) Erfinder: Thomas Magnete GmbH, 57562 Herdorf (DE); Hamm, Wolfgang, 57520 Mauden (DE); Mitterfellner, Lukas, 57299 Burbach (DE); Lotz, Ulrich, 57290 Neunkirchen (DE); Schlesinger, Uwe, 57290 Neunkirchen (DE)

(57) **Zusammenfassung**

2.1 Aufgabe: Im Zusammenwirken mit Schlauchpumpen soll die Schlauchklemme beim Schließen der Pumpe geöffnet und beim Öffnen der Pumpe geschlossen werden. Unabhängig von der Schlauchpumpe soll die Schlauchklemme im Anlieferzustand bei geöffnetem Schlauch ein Verschieben ermöglichen und nach dem Schließen des Schlauchs bei unverschieblicher Lage der Schlauchklemme den Schlauch geschlossen halten. Die Gestaltung soll der Schlauchklemme soll eine kostengünstige Herstellung in großen Stückzahlen ermöglichen.
2.2 Lösung: die Schlauchklemme umfasst in einem ersten Betriebszustand einen Schlauch **(2)** mittels eines ersten Durchbruchs **(3)** in einer Lasche **(8)** und umfasst in einem zweiten Betriebszustand mittels des ersten Durchbruchs **(3)** und mittels eines zweiten Durchbruchs **(4)** in einer zweiten Lasche **(9)** den Schlauch **(2),** wobei eine erste Sperre **(6)** und eine zweite Sperre **(7)** den Schlauch **(2)** in dem zweiten Durchbruch **(4)** halten und wobei ein Außenring **(5)** der Schlauchklemme über die Laschen und die Durchbrüche eine den Schlauch **(2)** verschließende Druckkraft auf den Schlauch **(2)** bewirkt, und die Schlauchklemme **(1)** umfasst in einem dritten Betriebszustand mittels des ersten Durchbruchs **(3)** und des zweiten Durchbruchs **(4)** den Schlauch, wobei die verschließende Druckkraft auf den Schlauch durch eine Verformung des Außenrings **(5)** mindestens teilweise aufgehoben ist.
2.3 Anwendung: Die erfindungsgemäße Schlauchklemme wird im Zusammenwirken mit einer Schlauchpumpe zur Förderung von pastösen Fluiden im Anwendungsfeld der medizinischen Pflege eingesetzt.

## Beschreibung

Die Erfindung betrifft eine Schlauchklemme entsprechend dem Oberbegriff des ersten Patentanspruchs. Schlauchklemmen dieser Art werden beispielsweise bei der medizinischen Pflege eingesetzt.

### Stand der Technik:

Aus den Druckschriften DE 3825201 A1, FR 2454577 und der noch nicht veröffentlichten DE 10 2013 014 217 sind beispielsweise Schlauchklemmen für die Verwendung in der Pflege bekannt.

Im Zusammenwirken mit Schlauchpumpen soll eine solche Schlauchklemme beim Schließen der Schlauchpumpe den Schlauch öffnen und beim Öffnen der Schlauchpumpe den Schlauch schließen und geschlossen halten. Unabhängig von der Schlauchpumpe soll die Schlauchklemme im Anlieferzustand unverlierbar, aber auf dem Schlauch verschieblich sein, und nach dem Schließen des Schlauchs bei gleichzeitiger Unverschieblichkeit den Schlauch ohne weitere Betätigung geschlossen halten.

Die bekannten Schlauchklemmen erfüllen entweder nicht alle genannten Teilaufgaben, oder sie sind unvertretbar aufwändig herzustellen.

Die Aufgabe der Erfindung besteht darin, alle oben genannten Teilaufgaben zu erfüllen und eine kostengünstige Herstellung der Schlauchklemmen in großen Stückzahlen zu ermöglichen.

Die Aufgabe wird durch die kennzeichnenden Merkmale des ersten Patentanspruchs in Verbindung mit den Unteransprüchen gelöst.

Im Auslieferungszustand des Schlauchs und der Schlauchklemme umfasst die Schlauchklemme den Schlauch mittels eines ersten Durchbruchs in einer ersten Lasche.

Nach einer ersten drückenden Betätigung umfasst die Schlauchklemme mittels des ersten Durchbruchs und mittels eines zweiten Durchbruchs in einer zweiten Lasche den Schlauch, wobei zwei Sperren den Schlauch in dem zweiten Durchbruch halten und wobei ein elastischer Außenring der Schlauchklemme über die Laschen und die Durchbrüche eine den Schlauch verschließende Druckkraft auf den Schlauch bewirkt.

Der Schlauch gelangt seitlich durch eine Passage in den zweiten Durchbruch, wenn der Außenring zusammengedrückt wird. Er kann durch diese Passage den zweiten Durchbruch aber nicht wieder verlassen, weil er durch die einseitig wirkenden Sperren zurückgehalten wird.

Nach dem Einlegen des Schlauchs in die Schlauchpumpe und nach dem Schließen der Schlauchpumpe wird der Außenring drückend verformt. Die Schlauchklemme umfasst weiterhin mittels des ersten Durchbruchs und des zweiten Durchbruchs den Schlauch, aber die Durchbrüche überlappen sich erheblich, dadurch ist die verschließende Druckkraft auf den Schlauch aufgehoben.

Wird die Schlauchpumpe wieder geöffnet, weitet sich der Außenring wieder auf und der Schlauch wird wieder verschlossen, weil der Außenring die beiden Durchbrüche so gegeneinander verschiebt, dass der Schlauch zusammengedrückt wird.

Nun kann der Schlauch mit der Schlauchklemme aus der Schlauchpumpe entnommen werden, er bleibt dabei verschlossen.

Soll die Schlauchklemme dann wieder geöffnet werden, kann dieses manuell durch ein Zusammendrücken des Außenrings erfolgen. Dabei überlappen sich die beiden Durchbrüche so, dass der Schlauch sich öffnen kann, und der Bediener spürt eine Gegenkraft, damit wird eine fehlerhafte Bedienung unwahrscheinlich.

Damit der Schlauch in der Schlauchklemme nicht verkantet, weist eine Lasche eine Ausnehmung auf, in die die andere Lasche seitlich eindringen kann.

Die Verformung des Schlauchs im geschlossenen Zustand wird durch eine Ausbuchtung des ersten Durchbruchs verstärkt und die Druckwirkung auf den Schlauch wird durch einen Steg am ersten Durchbruch , der in den zweiten Durchbruch seitlich eintaucht, verbessert.

Zur Verstärkung der Federkraft des Außenrings weist dieser elastische Stützkörper auf, die bei einem Zusammendrücken des Außenrings sich berühren und so ein nichtlineares Federmittel bilden.

Die Schlauchklemme ist einstückig durch Spritzgießen aus einem geeigneten Kunststoff hergestellt, vorzugsweise aus Polyketon oder Polyetherimid.

### Anwendung:

Die erfindungsgemäße Schlauchklemme wird im Zusammenwirken mit einer Schlauchpumpe zur Förderung von pastösen Fluiden im Anwendungsfeld der medizinischen Pflege eingesetzt.

### Bilder:

Fig. 1 zeigt die Schlauchklemme im Auslieferungszustand, der Schlauch ist offen.
Fig. 2 zeigt die Schlauchklemme in einem zweiten Betriebszustand, der Schlauch wird von den beiden Durchbrüchen umfasst und ist geschlossen.
Fig. 3 zeigt die Schlauchklemme in einem dritten Betriebszustand, die Schlauchklemme wird von einer äußeren Kraft zusammengedrückt und der Schlauch ist offen.

### Beispielhafte Ausführung

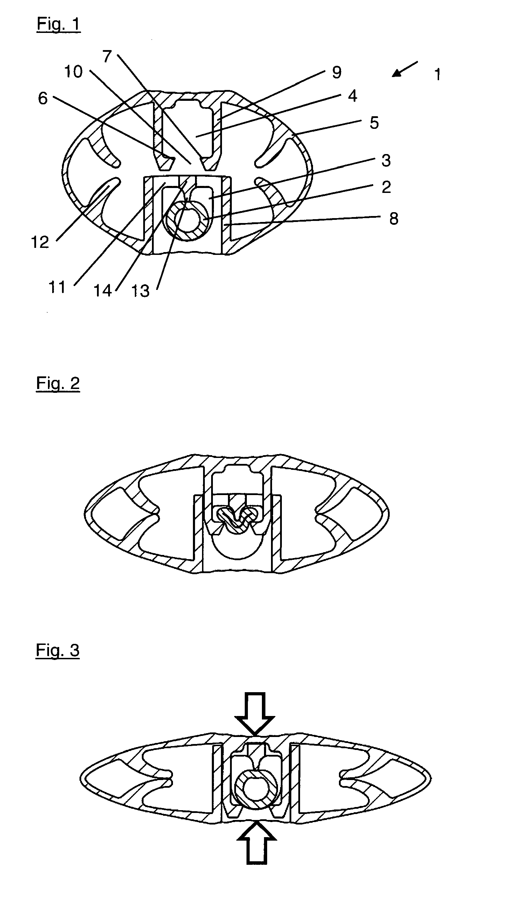

Eine Schlauchklemme **(1)** besteht aus einem elastischen Kunststoffmaterial und kann drei Betriebszustände einnehmen:
1. In einem ersten Betriebszustand gemäß Fig. 1 umfasst die Schlauchklemme einen Schlauch **(2)** mittels eines ersten Durchbruchs **(3)** in einer Lasche **(8).**
2. In einem zweiten Betriebszustand gemäß Fig. 2 umfasst die Schlauchklemme mittels des ersten Durchbruchs **(3)** und mittels eines zweiten Durchbruchs **(4)** in einer zweiten Lasche **(9)** den Schlauch **(2),** wobei eine erste Sperre **(6)** und eine zweite Sperre **(7)** den Schlauch **(2)** in dem zweiten Durchbruch **(4)** halten und wobei ein Außenring **(5)** der Schlauchklemme über die Laschen und die Durchbrüche eine den Schlauch **(2)** verschließende Druckkraft auf den Schlauch **(2)** bewirkt.
3. In einem dritten Betriebszustand gemäß Fig. 3 umfasst die Schlauchklemme **(1)** mittels des ersten Durchbruchs **(3)** und des zweiten Durchbruchs **(4)** den Schlauch, wobei die verschließende Druckkraft auf den Schlauch mindestens teilweise aufgehoben ist, weil der Außenring **(5)** durch eine äußere Kraft verformt ist.

Der erste Durchbruch **(3)** ist in einer ersten Lasche **(8)** angeordnet, die mit dem Außenring **(5)** verbunden ist.

Der zweite Durchbruch **(4)** in einer zweiten Lasche **(9)** angeordnet, die ebenfalls mit dem Außenring **(5)** verbunden ist.

Die erste Sperre **(6)** und die zweite Sperre **(7)** sind so an einer Passage **(10)** in dem zweiten Durchbruch **(4)** angeordnet, dass der Schlauch **(2)** bei einer erstmaligen drückenden Verformung des Außenrings **(5)** die Sperren passierend in den Durchbruch **(4)** eindringt, aber bei einer Entlastung des Außenrings **(5)** nicht wieder freigegeben wird, weil die Sperren ein Verlassen des Durchbruchs **(4)** durch die Passage **(10)** verhindern.

Vorzugsweise weist die erste Lasche **(8)** eine Ausnehmung **(11)** auf, in die die zweite Lasche **(9)** eindringt, wenn der Außenring **(5)** zusammengedrückt wird, und die erste Lasche **(8)** weist eine Ausbuchtung **(13)** auf. Ein Steg **(14)** überbrückt die Ausnehmung **(11)** und kann in die Passage **(10)** eindringen. Durch die Ausbuchtung **(13)** und insbesondere durch den Steg **(14)** wird die Verformung des Schlauchs in dem zweiten Betriebszustand verstärkt.

Der Außenring **(5)** weist vorteilhafterweise federnde Stützkörper **(12)** auf, die sich bei einem Zusammendrücken des Außenrings gegenseitig abstützen und so ein nichtlineares Federmittel bilden.

Die Schlauchklemme ist vorzugsweise aus einem der Kunststoffe Polyketon oder Polyetherimid hergestellt.

Die Verwendung der Schlauchklemme **(1)** erfolgt nach dem folgenden Verfahren:
1. Der Schlauch **(2)** wird so in einen ersten Durchbruch **(3)** der Schlauchklemme **(1)** eingeführt, dass er von dem Durchbruch **(3)** umfasst wird
2. Bei einem Zusammendrücken eines Außenrings **(5)** der Schlauchklemme **(1)** wird ein zweiter Durchbruch **(4)** in eine den Schlauch **(2)** umschließende Lage gebracht, weil der Schlauch **(2)** bei dem Zusammendrücken des Außenrings **(5)** eine Passage **(10)** mit einseitig wirkenden Sperren **(6)** und **(7)** im zweiten Durchbruch **(4)** passiert.
3. Bei einem Nachlassen der äußeren Druckkraft auf den Außenring **(5)** umschließt der zweite Durchbruch **(4)** den Schlauch weiter und die Kraft des Außenrings **(5)** bewirkt eine den Schlauch **(2)** verschließende Druckkraft auf den Schlauch **(2),** weil die einseitig wirkenden Sperren **(6)** und **(7)** den Schlauch nicht wieder freigeben.
4. Bei einem Zusammendrücken des Außenrings **(5)** wird die verschließende Druckkraft auf den Schlauch mindestens teilweise aufgehoben, weil die Durchbrüche sich stärker überlappen.

### Liste der Bezugszeichen

1. Schlauchklemme
2. Schlauch
3. Durchbruch
4. Durchbruch
5. Außenring
6. Sperre
7. Sperre
8. Lasche
9. Lasche
10. Passage
11. Ausnehmung
12. Stützkörper
13. Ausbuchtung
14. Steg

## Patentansprüche

1. Schlauchklemme **(1)** aus einem elastischen Kunststoffmaterial wobei
• die Schlauchklemme in einem ersten Betriebszustand einen Schlauch **(2)** mittels eines ersten Durchbruchs **(3)** in einer Lasche **(8)** umfasst
• und die Schlauchklemme **(1)** in einem zweiten Betriebszustand mittels des ersten Durchbruchs **(3)** und mittels eines zweiten Durchbruchs **(4)** in einer zweiten Lasche **(9)** den Schlauch **(2)** umfasst, wobei eine erste Sperre **(6)** und eine zweite Sperre **(7)** den Schlauch **(2)** in dem zweiten Durchbruch **(4)** halten und wobei ein Außenring **(5)** der Schlauchklemme über die Laschen und die Durchbrüche eine den Schlauch **(2)** verschließende Druckkraft auf den Schlauch **(2)** bewirkt
• und die Schlauchklemme **(1)** in einem dritten Betriebszustand mittels des ersten Durchbruchs **(3)** und des zweiten Durchbruchs **(4)** den Schlauch umfasst, wobei die verschließende Druckkraft auf den Schlauch wegen einer erheblichen Überlappung der Durchbrüche infolge einer Verformung des Außenrings **(5)** durch eine von außen wirkende Kraft mindestens teilweise aufgehoben ist.

2. Schlauchklemme nach Anspruch 1, **dadurch gekennzeichnet, dass** der erste Durchbruch **(3)** in einer ersten Lasche **(8)** angeordnet ist, die mit dem Außenring **(5)** verbunden ist.

3. Schlauchklemme nach Anspruch 1 oder 2, **dadurch gekennzeichnet, dass** der zweite Durchbruch **(4)** in einer zweiten Lasche **(9)** angeordnet ist, die mit dem Außenring **(5)** verbunden ist.

4. Schlauchklemme nach Anspruch 3, **dadurch gekennzeichnet, dass** die erste Sperre **(6)** und die zweite Sperre **(7)** so an einer Passage **(10)** in dem zweiten Durchbruch **(4)** angeordnet sind, dass der Schlauch **(2)** bei einer drückenden Verformung des Außenrings **(5)** die Sperren passierend in den zweiten Durchbruch **(4)** eindringt, aber bei einer Entlastung des Außenrings **(5)** nicht wieder freigegeben wird, weil die Sperren ein Verlassen des Durchbruchs **(4)** durch die Passage **(10)** verhindern.

5. Schlauchklemme nach einem der Ansprüche 3 bis 4, **dadurch gekennzeichnet, dass** die zweite Lasche **(9)** in eine Ausnehmung **(11)** der ersten Lasche **(8)** eingedrungen ist, wenn der Außenring **(5)** zusammengedrückt ist.

6. Schlauchklemme nach einem der Ansprüche 1 bis 5, **dadurch gekennzeichnet, dass** der Außenring **(5)** federnde Stützkörper **(12)** aufweist, die sich bei einem Zusammendrücken des Außenrings gegenseitig abstützen und so zusammen mit dem Außenring ein nichtlineares Federmittel bilden.

7. Schlauchklemme nach einem der Ansprüche 2 bis 6, **dadurch gekennzeichnet, dass** die erste Lasche **(8)** eine Ausbuchtung **(13)** aufweist, die bei einer Auffederung des Außenrings **(5)** die Verformung des Schlauchs verstärkt.

8. Schlauchklemme nach einem der Ansprüche 5 bis 7, **dadurch gekennzeichnet, dass** die Ausnehmung **(11)** der ersten Lasche **(8)** durch einen Steg **(14)** überbrückt wird, der in die Passage **(10)** der zweiten Lasche **(9)** eindringen kann.

9. Schlauchklemme nach einem der Ansprüche 1 bis 8, **dadurch gekennzeichnet, dass** die Schlauchklemme aus dem Kunststoff Polyketone hergestellt ist.

10. Schlauchklemme nach einem der Ansprüche 1 bis 8, **dadurch gekennzeichnet, dass** die Schlauchklemme aus dem Kunststoff Polyetherimid hergestellt ist.

11. Verfahren zur Verwendung einer Schlauchklemme **(1)** nach Anspruch 1 **dadurch gekennzeichnet,**
**dass** ein Schlauch **(2)** so in einen ersten Durchbruch **(3)** der Schlauchklemme **(1)** eingeführt wird, dass er von dem Durchbruch **(3)** umfasst wird
und **dass** bei einem Zusammendrücken eines Außenrings **(5)** der Schlauchklemme **(1)** ein zweiter Durchbruch **(4)** in eine den Schlauch **(2)** umschließende Lage gebracht wird, weil der Schlauch **(2)** bei dem Zusammendrücken des Außenrings eine Passage **(10)** mit einseitig wirkenden Sperren **(6)** und **(7)** im zweiten Durchbruch **(4)** passiert, und dass bei einem Nachlassen der Druckkraft auf den Außenring **(5)** der zweite Durchbruch **(4)** den Schlauch weiter umschließt und der Außenring **(5)** eine den Schlauch **(2)** verschließende Druckkraft auf den Schlauch **(2)** bewirkt, weil die einseitig wirkenden Sperren **(6)** und **(7)** den Schlauch nicht wieder freigeben
und **dass** bei einem folgenden Zusammendrücken des Außenrings **(5)** die verschließende Druckkraft auf den Schlauch mindestens teilweise aufgehoben wird.
